# EUROPEAN PATENT APPLICATION

(11) **EP 1 891 944 A1**
(43) Date of publication of application: **27.02.2008**
(21) Application number: 06291201.9
(22) Date of filing: 24.07.2006
(51) Int. Cl.: A61K 31/00, A61K 31/593, A61P 37/08, A61P 11/06, A61P 17/00

(54) **Use of Vitamin D3 agonist in a mammalian model for atopic diseases and of Vitamin D3 antagonists for the treatment of atopic diseases**

(71) Applicant: Association pour la recherche à l'IGBMC (ARI), 67000 Strasbourg (FR)
(72) Inventor: Chambon, Pierre, 67113 Blaesheim (FR); Metzger, Daniel, 67200 Strasbourg (FR); Li, Mei, 67100 Strasbourg (FR)
(74) Representative: Bredema

(57) **Abstract**

The present invention concerns a method for generating a human atopic disease-like phenotype, preferably an atopic dermatitis (AD)-like phenotype in a mammal comprising administrating to said mammal at least one compound selected in the group comprising the physiologically active vitamin D3 (1α,25 (OH)₂ D₃) and agonistic analogs thereof. The present invention also concerns a method for treating and/or preventing an atopic disease in a patient comprising administrating to said patient an effective amount of at least one vitamin D3 antagonist.

SEQUENCE LISTING

<110> ASSOCIATION POUR LA RECHERCHE A L'IGBMC (ARI)
CHAMBON, Pierre
METZGER, Daniel
LI, Mei

<120> methods for generating mammalian models of atopic diseases and screening
for their treatment

<130> 50632/EP

<160> 8

<170> PatentIn version 3.1

<210> 1
<211> 15
<212> DNA
<213> Mus musculus

<400> 1
aggacagcca gggct 15

<210> 2
<211> 15
<212> DNA
<213> Mus musculus

<400> 2
gagccagagg ggtca 15

<210> 3
<211> 13
<212> DNA
<213> Mus musculus

<400> 3
gggtcagggg aca 13

<210> 4
<211> 14
<212> DNA
<213> Mus musculus

<400> 4
agctcaacag gtca 14

<210>  5
<211>  15
<212>  DNA
<213>  Homo sapiens

<400>  5
agttctaaag gttca                                                        15


<210>  6
<211>  14
<212>  DNA
<213>  Homo sapiens

<400>  6
aggacaatgg gtat                                                         14


<210>  7
<211>  13
<212>  DNA
<213>  Homo sapiens

<400>  7
aggacacaag tca                                                          13


<210>  8
<211>  15
<212>  DNA
<213>  Homo sapiens

<400>  8
agcataatga ggtca                                                        15

1

## Description

### Field of the Invention

This invention relates to the field of atopic diseases, in particular to atopic dermatitis (AD).

### Background

Human atopic diseases are major health problems and comprise atopic dermatitis (AD), asthma, and allergic rhinitis (SPERGEL and PALLER, J. Allergy Clin. Immunol., vol. 112 (6 Suppl), p: S118-27, 2003).

Human atopic dermatitis (AD) is a chronic skin inflammatory disease with a strong genetic component that affects children (10-20%) and adults (1-3%) (LEUNG et al., J. Clin. Invest., vol.113, p:651-657, 2004). Atopic dermatitis results notably in skin eczematous-like lesions with xerosis and pruritus, associated with a skin inflammatory infiltrate mainly composed of CD4+ T helper type 2 (Th2) cells, dendritic cells, eosinophils and mast cells, and systemic abnormalities including elevated serum IgE and IgG levels, as well as blood and tissue eosinophilia.

Asthma is a disorder characterized by lung inflammation (with elevated eosinophils, Th2 cells and macrophages, as well as Th2-type cytokines), intermittent reversible airway obstruction, airway hyperreactivity (AHR), excessive mucus production, and elevated serum levels of IgE and Th2-type cytokines. It affects approximately 5% of the population in the Western world and its incidence is increasing dramatically in developed nations (RENAULD, J. Clin. Pathol., vol.54(8), p:577-89, 2001; ELIAS et al., J. Clin. Invest., vol.111(3), p:291-7, 2003). It is noteworthy that approximately half of AD patients will develop asthma at later stages of their life, often associated with severe AD (SPERGEL and PALLER, 2003, abovementioned).

Nuclear receptors (NRs) belong to a superfamily of ligand-dependent transcriptional regulators (LAUDET, & GRONEMEYER, The nuclear receptor : factsbook, Academic Press, San Diego, 2002; MANGELSDORF et al., Cell, vol.83, p :835-839, 1995). Within this superfamily, Retinoid X Receptors (RXRs) α, β and γ play a key role through heterodimerization with some 15 NR partners, e.g. Retinoic Acid Receptors (RARs) α, β and γ, Vitamin D Receptor (VDR), Peroxisome Proliferator-Activated Receptors (PPARs) and liver X receptors (LXRs).

The inventors have previously reported (LI et al., Proc. Natl. Acad. Sci. U S A, vol.102, p :14795-14800, 2005) that selective ablation of RXRα and RXRβ in adult mouse epidermal keratinocytes (RXRαβ^{ep-/-} mice) triggers a skin and systemic phenotype similar to human atopic dermatitis (AD). In fact, these mice exhibit the major features of the human AD syndrome that includes (i) skin eczematous-like lesions with xerosis and pruritus, associated with a skin inflammatory infiltrate mainly composed of CD4+ T helper type 2 (Th2) cells, dendritic cells, eosinophils and mast cells, and (ii) systemic abnormalities including elevated serum IgE and IgG levels, and blood and tissue eosinophilia. The inventors also established that expression of the cytokine thymic stromal lymphopoietin (TSLP), known to be produced in epidermal keratinocytes of AD patients (SOUMELIS et al., Nat. Immunol., vol.3, p :673-680, 2002), is rapidly induced in keratinocytes of RXRαβ^{ep-/-} mice. Furthermore, the inventors showed that K14-TSLP transgenic mice overexpressing TSLP in keratinocytes exhibit an AD-like phenotype similar to that of RXRαβ^{ep-/-} mice (LI *et al.,* 2005, abovementioned), demonstrating that TSLP can act as an initiating cytokine at the top of a chain of immunological events that lead to an AD-like phenotype. In addition the inventors have recently shown that ablation of the TSLP gene in mice prevents the generation of an AD-like phenotype (unpublished data), demonstrating that TSLP expression is both sufficient and necessary for generating an AD in the mouse. In keeping with these results others have shown that in human asthmatic airways TSLP expression is increased and correlated with both the expression of Th2-attracting chemokines and with disease severity. Moreover, lung-specific expression of a TSLP transgene induced asthma-like syndrome, whereas, in contrast, mice lacking the TSLP receptor (TSLPR) failed to develop asthma in response to inhaled antigens (LIU, J. Exp. Med., vol.203, p :269-273, 2006 ; ZHOU et al.., Nat. Immunol., vol.6, p :1047-1053, 2005 ; YOO et al., J. Exp. Med., vol.202, p :541-549, 2005; AL-SHAMI et al., J. Exp. Med., vol.202, p :829-839, 2005; YING et al., J. Immunol., vol.174(12), p:8183-8190, 2005). The expression of TSLP in epidermis and lung epithelium is therefore necessary and sufficient to trigger in the mouse an AD-like phenotype and an asthma-like syndrome, respectively.

However, the molecular mechanism underlying the induction of TSLP expression remains unknown, thus precluding the development of novel mouse models in which the generation of human-like atopic diseases would allow screening for drugs which could be used for human atopic disease treatment through interference with the physiological mechanism that controls TSLP expression.

### Summary of the Invention

This invention provides a method for generating a human atopic disease-like phenotype in a mammal comprising the step (i) of administrating to said mammal at least one compound selected in the group comprising the physiologically active vitamin D3 [1α,25 (OH)₂ D₃] and its agonistic analogs.

In a preferred embodiment, said step (i) further comprises administrating to said mammal at least one compound selected in the group comprising natural and synthetic Retinoic Acid Receptor (RAR) agonists, preferably a RARγ-selective agonist.

In a still preferred embodiment, said method further comprises the step (ii) of assessing the generation of atopic disease in said mammal.

In another preferred embodiment, said method further comprises the step (iii) of administrating to said mammal a compound that can be useful for treating and/or preventing atopic disease.

In still another preferred embodiment, said method further comprises the step (iv) of analyzing the human atopic disease-like phenotype of the mammal with or without the administration of the compound that has been identified to be possibly useful for treating and/or preventing a human atopic disease.

In still another preferred embodiment, said method further comprises the step (v) of selecting the compounds that revert and/or prevent the human atopic disease-like phenotype.

Advantageously, said compound that can be useful for treating and/or preventing an atopic disease is selected in the group comprising vitamin D3 antagonists and RAR antagonists.

This invention also provides a method for treating and/or preventing an atopic disease in a patient comprising the step (i) of administrating to said patient an effective amount of at least one vitamin D3 antagonist.

In a preferred embodiment, said method for treating and/or preventing atopic disease further comprises the step (ii) of administrating to said patient an effective amount of at least one RAR antagonist.

### Detailed Description

As RXR/NR heterodimers in which an agonistic ligand is not bound to the NR partner can act as transcriptional repressors (PERISSI and ROSENFELD, Nat. Rev. Mol. Cell. Biol., vol.6, p :542-554, 2005), the inventors have examined whether TSLP expression could be modified by NR agonists.

Among the tested NR agonists, the active derivatives of vitamin D3 and vitamin A have been considered.

Vitamin D3 is a secosteroid, which is involved in the control of a wide variety of biological processes in higher animals. These processes include maintenance of calcium homeostasis, immunomodulation and selected cell differentiation. Vitamin D3, itself, is biologically inactive. However, metabolism of vitamin D3 to the biologically active compound 1,25-Dihydroxyvitamin D3 [1α,25-(OH)₂D₃] is responsible for the wide array of biological responses which are observed as part of the vitamin D endocrine system.

Vitamin A (retinol) is an essential component of the diet. Both clinical and experimental approaches have shown that vitamin A derivatives (retinoids) are necessary for normal growth, vision, and maintenance of numerous tissues, reproduction and overall survival (BLOMHOFF, Nutr. Rev., vol.52(1 Pt 2), p:S13-23, 1994; SPORN et al., The retinoids. Biology, Chemistry and Medecine., New York: Raven 1994). Vitamin A is also known to play an important role in immune responses (STEPHENSEN, Annu. Rev. Nutr., vol.21, p:167-92, 2001). The active derivative of Vitamin A is retinoic acid (RA). Experimentally one can manipulate retinoid levels in vivo by providing vitamin A deficient diets or by administering RA or its synthetic agonistic analogs.

Recently it has been suggested that vitamin D and vitamin A could possibly be used to treat AD patients (WORM, Curr. Opin. Investig. Drugs, vol.3, p :1596-1603, 2002 ; LEHMANN et al., Exp. Dermatol., vol.13 (Suppl 4), p :11-15, 2004 ; ZASLOFF, J. Invest. Dermatol., vol. 125, p :xvi-xvii, 2005 ; ZASLOFF, Nat. Med., vol. 12, p :388-390, 2006).

On the contrary, the inventors have now established that administration of active derivatives of these vitamins generates an AD-like phenotype in the mouse, and therefore may exacerbate AD in patients, by promoting expression of TSLP in epidermis.

Consequently, a first object of the present invention concerns a method for generating a human atopic disease-like phenotype in a mammal comprising the step (i) of administrating to said mammal at least one compound selected in the group the physiologically active vitamin D3 [1α,25 (OH)₂ D₃], and its agonistic analogs.

Preferably, said mammal is non-human, and most preferably a mouse.

As used herein, the term "atopic disease" refers to a disease selected in the group comprising atopic dermatitis (AD), asthma and allergic rhinitis.

In one embodiment, said atopic disease is atopic dermatitis.

Preferably, said compound is administrated to the skin of said mammal, preferably to the ear skin.

In addition, said method further comprises the step of (ii) assessing the generation of a human atopic-disease phenotype, preferably an AD-like phenotype. Such assessing step can be realized by external skin aspect observation, skin histological examination (i.e., presence of eosinophils, mast cells and dendritic cells), analyzing TSLP and Th2 type cytokine expression in skin (e.g., by northern blots and quantitative RT-PCR (Q-PCR) for RNA transcripts, or by western blot and immunohistochemistry for proteins), and analyzing TSLP and IgE serum levels (e.g., by ELISA), and blood eosinophilia.

The term "physiologically active vitamin D3 (1α,25 (OH)₂ D₃)" as used therein, refers to the 1,25-Dihydroxyvitamin D3.

Vitamin D3 agonistic analogs are known from one of skill in the art and include, as non-limiting examples, 1α,18,25-(OH)₃D₃, 23-(m-(Dimethylhydroxymethyl)-22-yne-24,25,26,27(teranor)-1α-OH)₂D₃, 1α,25-Dihydroxy-trans-Isotachysterol(1,25-trans-Iso-T), (1S,3R,6S)-7,19-Retro-1,25-(OH)₂D₃, (1S,3R,6R)-7,19-Retro-1,25-(OH)₂D₃, 22-(p-(Hydroxyphenyl)-23,24,25,26,27-pentanor-D₃, 22-(m-(Hydroxyphenyl)-23,24,25,26,27-pentanor-D₃ described in PCT application WO 95/17197, 26, 27-cyclo-22-ene-1α,24S-dihydroxyvitamin D3 (MC903), 1(S),3(R)-dihydroxy-20(R)-(5'-ethyl-5'-hydroxy-hepta-1'(E),3'(E)-dien-1'-yl)-9,10-secopregna-5(Z),7(E),10(19)-triene (EB1089), and 1α,25-(OH),-20-epi-22-oxa-24,26,27-trishomovitamin D (KH1060) described in CARLBERG et al. (J. Steroid. Biochem. Mol. Biol., vol.51, p :137-142, 1994), and 1R,25-dihydroxy-21-(3-hydroxy-3-methylbutyl)vitaminD₃ described in CARLBERG et al. (J. Cell Biochem., vol.88, p :274-281, 2003).

Vitamin D3 is also implicated in calcium homeostasis and its administration to a mammal may result in hypercalcemia.

Advantageously, said vitamin D3 agonistic analog is a low-calcemic vitamin D3 agonistic analog. Such low-calcemic vitamin D3 agonistic analogs are known from one of skill in the art and include, as non-limiting examples, a low-calcemic vitamin D3 synthetic agonistic analog selected in the group comprising 26, 27-cyclo-22-ene-1α,24S-dihydroxyvitamin D3 (MC903), 1 (S),3(R)-dihydroxy-20(R)-(5'-ethyl-5'-hydroxy-hepta-1'(E),3'(E)-dien-1'-yl)-9,10-secopregna-5(Z),7(E),10(19)-triene (EB1089), 1α,25-(OH),-20-epi-22-oxa-24,26,27-trishomovitamin D (KH1060), and 1R,25-dihydroxy-21-(3-hydroxy-3-methylbutyl)vitaminD₃.

The inventors have demonstrated that TSLP, which acts as an initiating cytokine at the top of a chain of immunological events leading to an AD-like phenotype, is induced by the administration of 1α,25 (OH)₂ D₃ or of MC903 to ear skin.

Therefore, said vitamin D3 agonistic analog has to induce TSLP expression and corresponds, as an example, to 26, 27-cyclo-22-ene-1α,24S-dihydroxyvitamin D3 (MC903).

One of skill in the art can simply determine the effective amount of vitamin D3 or agonistic analogs thereof to be administrated in order to induce an AD-like phenotype in view of its general knowledge and of the protocols described in the examples.

As an example, such effective amount for vitamin D3 is comprised in the range of 0.025 nmole to 4 nmoles per cm² of skin, preferably in the range of 0.1 nmole to 0.5 nmole per cm² of skin.

As an example, such an effective amount for the MC903 vitamin D3 agonistic analog is comprised in the range of 0.025 nmole to 5 nmoles per cm² of skin, preferably in the range of 0.5 nmole to 2.5 nmoles.

The inventors have also demonstrated that topical administration of a RAR agonist, or preferably a RARγ-selective agonist, boosts the TSLP increase induced by la,25 (OH)₂ D₃ administration.

In another embodiment, the step (i) further comprises administrating to said mammal of at least one compound selected in the group comprising natural and synthetic Retinoic Acid Receptor (RAR) agonists.

Natural and synthetic RAR agonists are well known from one of skill in the art and include, as examples, retinoic acid (RA) as a natural RAR agonist, and synthetic RAR agonists such as the RARγ-selective racemic mixture of R- and S-3-fluoro-4-[2-hydroxy-2-(5,5,8,8-tetramethyl-5,6,7,8,-tetrahydro-naphthalen-2-yl)-acetulamino]-benzoic acid (BMS961; BOURGUET & GERMAIN et al., Trends Pharmacol. Sci. , vol.21(10), p: 381-8, 2000), the pan-RAR agonist (R)-3-Fluoro-4-[2-hydroxy-2-(5,6,7,8-tetrahydro-5,5,8,8-tetramethylnaphthalen-2-yl)-2-acetylamino]benzoic Acid (BMS270394; KLAHOLZ et al., Proc. Natl. Acad Sci. U S A, vol.97, p: 6322-6327, 2000 ; KLAHOLZ et al., J. Mol. Biol., vol.302, p : 155-170, 2000), and the pan-RAR agonist (E)-4-[2-(5,6,7,8-Tetrahydro-5,5,8,8-tetramethylnaphthalen-2-yl)prop-l-en-l-yl]benzoic Acid (TTNPB; THACHER, VASUDEVAN et al.., Curr. Pharm. Des., vol.6(1), p: 25-58, 2000).

One of skill in the art can simply determine the effective amount of natural or synthetic RAR agonist to be administrated with vitamin D3 or agonistic analog thereof in order to induce an AD-like phenotype, in view of his general knowledge and of the protocols described in the examples.

As an example, such effective amount for BMS961 is comprised in the range of 0.025 nmole to 5 nmoles per cm² of skin, preferably in the range of 0.5 nmole to 2.5 nmoles.

In another preferred embodiment, said administration step corresponds to a daily administration and is maintained for a sufficient period to induce an atopic disease, preferably an atopic dermatitis.

As an example said sufficient period to induce an atopic disease, preferably an atopic dermatitis, corresponds to a daily administration for a period comprised between three days and one month, and preferably between 10 and 20 days, and can be resumed at any time thereafter.

In still another preferred embodiment, the method of the invention further comprises the step (iii) of administrating to said mammal a compound that could be useful for treating and/or preventing an atopic disease. Thus, the method of the invention enables to identify compounds that could be useful for treating and/or preventing an atopic disease, preferably atopic dermatitis (AD).

As used herein, "treating and/or preventing" an atopic disease means that symptoms of atopic disease are prevented, reduced or inhibited after the administering of said compound.

The administrating steps of (i) a compound selected in the group comprising the physiologically active vitamin D3 and its agonistics analogs, and of (iii) a compound that could be useful for treating and/or preventing an atopic disease can be realized simultaneously or successively.

The term "compound that could be useful for treating and/or preventing an atopic disease", as used herein, refers to any compound such as a polypeptide, an oligonucleotide, a polysaccharide, a vitamin D3 antagonist or a RAR antagonist.

Advantageously, said compound is a vitamin D3 antagonist or an RAR antagonist, and more preferably a vitamin D3 antagonist.

More advantageously, said compound is a combination of a vitamin D3 antagonist and of RAR antagonist.

Preferably, said at least one vitamin D3 antagonist and at least one RAR antagonist are administrated simultaneously or successively.

Vitamin D3 antagonists are well known from one of skill in the art and include, as examples, the vitamin D3 antagonistic analogs selected in the group comprising 14-Epi-1,25-(OH)₂D₃, 14-Epi-1,25-(OH)₂-Pre-D₃, 1,25-(OH)₂-7,8-cis-D₃, 1,25-(OH)₂-5,6-trans-7,8-cis-D₃ described in PCT application WO 95/17197, the vitamin D3 antagonists described in PCT application WO 02/15894, and butyl-(5Z,7E,22E)-(1S,3R,24R)-1,3,24-trihydroxy-26,27-cyclo-9,10-secocholesta-5,7,10(19),22-tetraene-25-carboxylate (ZK159222), (23S)-25-dehydro-1α(OH)D3-26,23-lactone (TEI 9647), and ZK168281 as described in CARLBERG (2003, abovementioned).

Preferably, said vitamin D3 antagonistic analog selectively antagonizes the effect of the physiologically active vitamin D3 on the immune system.

RAR antagonists are also well known from one of skill in the art and include, as examples, Pan-RAR antagonists selected in the group comprising 4-(6-Methoxyethoxymethoxy-7-adamantyl naphthalen-2-yl)benzoic Acid (CD2665; SZONDYet al., Mol. Pharmacol., vol.51(6), p: 972-82, 1997), 4-[2-(5,6-dihydro-5,5-dimethyl-8-p-tolylnaphthalen-2-yl)ethynyl]benzoic acid (AGN193109; KLEIN et al., J. Biol. Chem., vol.271(37), p: 22692-6, 1996) and (E)-4-[2-[5,6-Dihydro-5,5-dimethyl-8-(2-phenylethynyl)naphthalene-2-yl]ethen-1-yl]benzoic Acid (BMS493; GERMAIN et al., Nature, vol.415(6868), p: 187-92, 2002).

Preferably, said RAR antagonist is a RARγ-selective antagonist.

In a preferred embodiment, said method that allows the identification of a compound that can be useful for treating and/or preventing atopic disease further comprises the step (iv) of comparing the atopic disease phenotype of the mammal with or without the administration of the compound that can be useful for treating and/or preventing inflammatory atopic disease.

In a still preferred embodiment, said method that allows the identification of a compound that can be useful for treating and/or preventing atopic disease further comprises the step (v) of selecting the compounds that revert and/or prevent the atopic disease phenotype, preferably the AD-like phenotype.

The selected compounds may be then further tested for their toxicity and/or their ability to prevent or treat atopic disease in other animal models.

In still another preferred embodiment, the atopic disease is asthma.

Preferably the compound selected in the group comprising the physiologically active vitamin D3 and its agonistic analogs and eventually compounds that can be useful for treating and/or preventing asthma are administrated to lungs. Methods for administrating a compound to the lungs are well known from one of skill in the art and include aerosol inhalation.

Advantageously, said method further comprises the step of (ii) assessing the generation of asthma. Such assessing step can be realized by lung histopathological examination (e.g., identification of lung inflammation), analysis of bonchoalveolar lavage (BAL) fluid, lung TSLP and Th2-type cytokine expression, and by physiological tests of lung function (e.g. measurement of airway hyperresponsiveness as described in RANGASAMY et al. (J. Exp. Med., vol.202(1), p: 47-59 2005), and in Animal Models of Airway Sensitization (Unit 15.18 in Current Protocols in immunology, John Wiley & Sons, Inc., 1999)).

A second object of the present invention concerns a method for treating and/or preventing an atopic disease in a patient comprising the step of administrating to said patient an effective amount of at least one vitamin D3 antagonist.

The term "patient" as used herein refers to a mammal, preferably to a human.

Preferably, said atopic disease is atopic dermatitis (AD) or asthma, and more preferably atopic dermatitis (AD).

More preferably, said method further comprises the step of administrating to said patient at least one RAR antagonist.

The at least one vitamin D3 antagonist and at least one RAR antagonist can be administrated simultaneously or successively.

The at least one vitamin D3 antagonist with or without at least one RAR antagonist can be formulated into pharmaceutical compositions (also called "medicaments") comprising a pharmaceutically acceptable carrier. Formulation of pharmaceutical compositions of the invention are within the skill in the art, for example as described in Remington's Pharmaceutical Science, 17th ed., Mack Publishing Company, Easton, Pa. (1985), the entire disclosure of which is herein incorporated by reference.

According to the present invention, an "effective amount" of at least one vitamin D3 antagonist and possibly of at least one RAR antagonist is one, which is sufficient to achieve a desired biological effect, in this case the prevention or reversion of an atopic disease. It is understood that the effective dosage will be dependent upon the age, sex, health, and weight of the recipient, kind of concurrent treatment, if any, frequency of treatment, and the affinity of said antagonist for its receptor.

A third object of the present invention concerns a use of a composition comprising at least one vitamin D3 antagonist analog for the manufacture of a medicament for treating and/or preventing an atopic disease in a patient.

Preferably, said atopic disease is atopic dermatitis (AD) or asthma, and more preferably atopic dermatitis (AD).

More preferably, the composition further comprises at least one RAR antagonist.

The invention will now be illustrated by the following non-limiting examples.

### EXAMPLES

### 1) Topical application of 1α,25-(OH)2D3 (the physiologically active vitamin D3) or of its low-calcemic analog MC903 induces TSLP expression in epidermal keratinocytes

As RXR/NR heterodimers in which an agonistic ligand is not bound to the NR partner can act as transcriptional repressors (PERISSI and ROSENFELD, 2005, abovementioned), we examined whether TSLP expression could be induced by a variety of agonistic ligands of NRs known to heterodimerise with RXRs.

Four nmoles of ligands dissolved in ethanol were topically applied to whole ears of 6-8-week-old female CD1 wildtype (WT) mice for 4 consecutive days (D1 to D4), and TSLP RNA levels were determined on day 5.

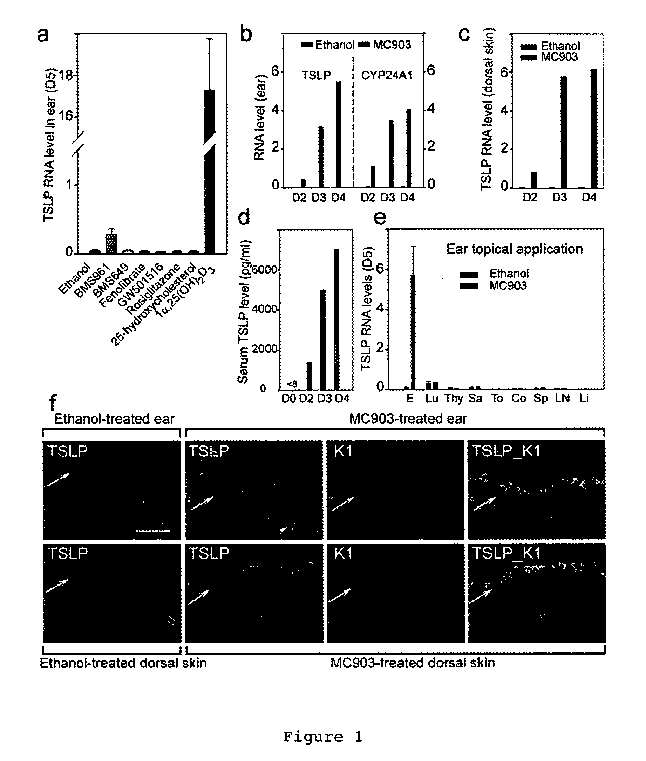

Figure 1a shows the TSLP RNA level (determined by Q-PCR) in ear epidermal keratinocytes at day 5 (D5) after skin topical application of ethanol, BMS961, BMS649, fenofibrate, GW501516, Rosiglitazone and 1α,25(OH)₂D3.

The results show that topical application of selective agonists for RXRs (BMS649, also known as SR11237; LEHMANN et al., Science, vol.258, p:1944-1946, 1992), PPARα (Fenofibrate) (INOUE et al., Biochem. Biophys. Res. Commun., vol.290, 131-139, 2002), PPARβ (GW501516, OLIVER et al., Proc. Natl. Acad. Sci. USA, vol.98, p:5306-5311, 2001), PPARγ (Rosiglitazone; LEHMANN et al., J. Biol. Chem., vol.270, p:3406-3410, 1997) and LXRs (25-hydroxycholesterol; FOWLER et al., J. invest. Dermatol., vol.120, p: 246-255, 2003) had no effect on TSLP expression (determined by Q-PCR; see Fig. 1a). In marked contrast, application of 1α,25-(OH)2D3 led to a dramatic increase (>300-fold) in TSLP transcripts at D5, whereas they were modestly, but significantly increased (5-fold) upon application of a RARγ-selective agonist (BMS961 ; CHAPELLIER et al., Embo J., vol.21, p :3402-3413, 2002 ; see Fig. 1a).

As, at this dose, 1α,25-(OH)2D3 application resulted in hypercalcemia and death of the mice, we applied its analog MC903 (calcipotriol, Dovonex; CARLBERG, 2003, abovementioned) dissolved in ethanol, that exhibits a low calcemic activity and is used for psoriasis treatment (KRAGBALLE and IVERSEN, Dermatol. Clin., vol.11, p : 137-141, 1993). MC903 and ethanol (vehicle) were applied to WT mouse right and left ears, respectively. Two shaved areas (1cm² each) of dorsal skin were also treated with MC903 or ethanol.

Figure 1b shows the TSLP and CYP24A1 RNA levels (determined by Q-PCR) in ethanol-treated left ear and MC903-treated right ear at day 2, 3 and 4 (D2, D3 and D4) after skin topical application.

Figure 1c shows the TSLP RNA levels (determined by Q-PCR) in ethanol-treated and MC903-treated dorsal skin (c) at D2, D3 and D4. Figure 1 d shows the serum TSLP levels (pg/ml) at D0, D2, D3 and D4 (these data are representative of three independent experiments).

The results show that one day after the first application (D2), TSLP RNA levels were increased in right ears, and further increased on D3 and D4, whereas no increase occurred in left ears (see Fig. 1b, left panel). Transcripts of *CYP24A1,* a 1α,25-(OH)2D3-inducible gene (JONES et al., Physiol. Rev., vol.78, p :1193-1231, 1998), were increased upon MC903 application, as expected (see Fig. 1b, right panel). TSLP RNA was also increased in MC903-treated dorsal skin (see Fig.1c), and serum TSLP levels were increased at D2-D4, while undetectable at D0 (before treatment, see Fig. 1d). Increasing doses of MC903 (0.4, 1 or 4 nmoles per ear) led to a dose-dependent increase of TSLP transcripts, which was similarly observed with other low-calcemic analogs of 1α,25-(OH)2D3, including EB1089 and KH1060 (CARLBERG, 2003, abovementioned ; CARLBERG *et al. ,* 1994, abovementioned ; and our data not shown).

To examine whether MC903-induced expression of TSLP was skin-restricted, various other organs were analyzed at D5.

Figure 1e shows the TSLP RNA levels (determined by Q-PCR) at D5 of mice topically treated by ethanol or MC903 on ears : in ear (E), lung (Lu), thymus (Thy), salivary gland (Sa), tongue (To), colon (Co), spleen (Sp), lymph node (LN) and liver (Li).

The results show that no increase in TSLP transcripts was observed in these organs, with the exception of ears (see Fig. 1e).

Figure 1f shows the expression of TSLP and keratin 1 (K1) by immunochemistry at D4 in sections of ear (upper panels) and dorsal skin (lower panels) of mice topically treated with ethanol or MC903, as indicated. The white arrowhead points to autofluorescent erythrocytes, and white arrows point to the dermal/epidermal junction. (Scale bar, 50 µm).

Immunohistochemistry (IHC) shown in Fig.1f did not reveal TSLP expression in epidermis and dermis of ethanol-treated ear or dorsal skin, whereas it was readily detected at D4 upon MC903 treatment (see Fig. 1f; ear skin: upper panel; dorsal skin: lower panel). Double IHC for TSLP and keratin 1 (K1, used as a suprabasal keratinocyte marker), showed that TSLP was mainly located in these keratinocytes in both MC903-treated ear and dorsal skin, while it could also be detected at a lower level in basal keratinocytes (expressing keratin 14) (see Fig. 1f, and data not shown).

### 2) Topical application of MC903 triggers an AD-like syndrome

As TSLP expression appears to be critically involved in the initiation of AD-like dermatitis in the mouse (LI *et al.,* 2005, abovementioned, YOO *et al.,* 2005, abovementioned), we investigated whether a MC903 long-term treatment could induce an AD-like phenotype.

MC903 (4 nmoles) was daily applied for 16 days to ears of 6-8-week-old female CD1 wildtype (WT) mice. No hypercalcemia or overall health impairment and weight loss was observed. Ethanol application did not cause any change in ear appearance, whereas reddening and swelling, that worsened with time, were observed from D5 on MC903-treated ears (not shown).

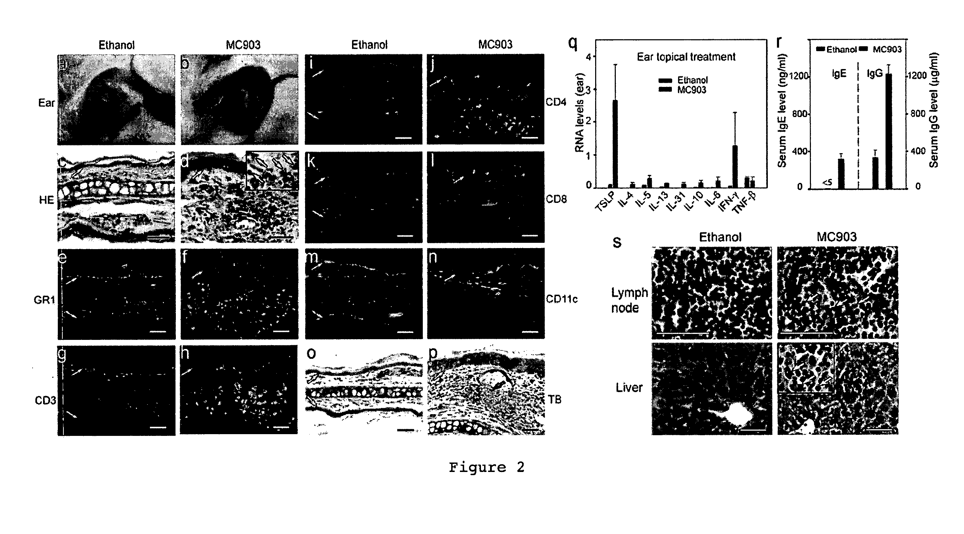

Figure 2 shows the effect of ear topical treatment with MC903 or ethanol. White arrows point to the dermal/epidermal junction.

Figures 2a and b show the ear appearance at day 17 after ethanol and MC903 treatment, respectively.

Figures 2c and d show hematoxylin and eosin-stained (HE) ear sections of ethanol- and MC903-treated mice at day 17, respectively. Eosinophil-selective cytoplasmic red staining is pointed by yellow arrows in the inset of (d).

Figures 2 e to n show the result of IHC performed on ear sections from ethanol- or MC903-treated mice at D17, with antibodies against GR1 (e and f), CD3 (g and h), CD4 (i and j), CD8 (k and 1) and CD11c (m and n). Yellow color corresponds to staining of antibodies, whereas blue corresponds to DAPI staining of nuclei.

Figures 2 o and p show the Toluidin blue (TB)-staining of ear sections. Red arrows point to one of the mast cells with intense blue color in the dermis.

Figure 2 q shows cytokine RNA levels (determined by Q-PCR) in ethanol- and MC903-treated ears at D17. The results show the results of ELISA experiments assessing the serum IgE and IgG levels in ethanol- and MC903-treated mice at D17. Figure 2 s show the hematoxylin and eosin-stained sections of ear draining lymph node and liver of ethanol- and MC903-treated mice at D17. Yellow arrows point to three of many eosinophils (red cytoplasmic staining) in sections of lymph node and liver of MC903-treated mice. (Scale bars, 50 µm).

The results shows that at D17, these ears were red, scaly, swollen and crusted (see Fig. 2, compare a and b), while frequent ear scratching (not shown) suggested a pruritus. Histological analysis revealed epidermal hyperplasia and a heavy dermal cell infiltrate, in which numerous eosinophils were easily identified upon hematoxylin/eosin staining (see Fig.2d and inset). Their identity was confirmed with eosinophil-selective Luna's staining (not shown). In contrast, no eosinophils were found in ethanol-treated ears (see Fig. 2c). IHC with an anti-GR1 antibody (recognizing granulocytes and monocytes) revealed a large number of positive cells in dermis, of which eosinophils but not neutrophils were a major component (see Fig. 2f, and data not shown). Numerous T lymphocytes (CD3+) were observed in MC903-treated dermis (see Fig. 2h), whereas only few resident T lymphocytes could be detected in ethanol-treated ears (see Fig. 2g). Most of the infiltrated T cells were CD4+ helper T cells (see Fig. 2j), and only a few CD8+ cytotoxic T cells were found (see Fig. 21). A large increase in CD11c+ dermal dendritic cells was also observed in MC903-treated ears (see Fig. 2 m and n), whereas mast cells were 4-fold increased in the dermis (see Fig. 2 o and p, and data not shown).

As topical application of 1α,25-(OH)2D3 at a dose of 4 nmoles per ear resulted in mouse death within 7 days, WT mice were also treated every other day with a dose of 0.25 nmole 1α,25-(OH)2D3 per ear, to examine whether it would result in a skin inflammation similar to that generated with MC903.

At this dose, TSLP expression was significantly induced at D18, and an inflammatory infiltrate comprising CD4+ T lymphocytes, dendritic cells, eosinophils and mast cells could also be observed (data not shown).

Taken together, these data indicated that the inflammatory cell infiltrate observed in skin of MC903- and 1α,25-(OH)2D3-treated ears had the characteristics of an AD-like skin inflammation (LI *et al.,* 2005, abovementioned). This was fully supported by Q-PCR analysis of cytokine RNA expressed in MC903-treated ears. At D16, TSLP transcripts were markedly increased (see Fig. 2q), and Th2-type cytokine transcripts (IL-4, IL-5, IL-13, IL-31, IL10 and IL-6) (LI *et al.,* 2005, abovementioned) were all significantly increased (see Fig. 2q). Expression of the Th1-type cytokine IFN-γ was also enhanced, whereas that of TNF-β, another Th1-type cytokine, was unchanged. Importantly, this cytokine expression profile, which is essentially that of a Th2-type inflammation, was similar to those observed in skins of RXRαβ ^{ep-/-} and K14-TSLP transgenic mice (LI *et al.,* 2005, abovementioned), indicating that it was most probably due to enhanced TSLP production in keratinocytes. Systemic abnormalities, including elevated serum IgE and IgG levels, associated with blood and tissue eosinophilia, have been observed in RXRαβ^{ep-/-} and K14-TSLP mice, exhibiting similarities to those observed in AD patients (LI *et al.,* 2005, abovementioned). Serum IgE and IgG levels were increased in mice to which MC903 was topically applied for 16 days on ears (see Fig. 2r). Moreover, at D16, MC903-treated mice exhibited an increased number of eosinophils in ear-draining lymph nodes, liver and spleen (see Fig. 2s, and data not shown). Differential blood cell counts also revealed a marked increase in eosinophils in MC903-treated mice (693±220 cells/µl, versus 204±134 cells/µl in ethanol-treated mice). Thus, MC903 topical application leads to a skin and systemic phenotype mimicking that of human AD.

### 3) Both keratinocytic VDR and RXR are required for induction of TSLP expression and generation of an AD-like skin inflammation upon MC903 treatment

To investigate whether the MC903-induced TSLP expression and appearance of an AD-like skin inflammation were mediated through VDR, MC903 was topically applied on ears of "floxed" VDR control (CT) mice (VDR^{L2/L2} mice, in which both VDR alleles bear LoxP sites) and of their VDR^{ep-/-} littermates [K14-Cre^{(tg/0)}/VDR^{L2/L2} mice in which the VDR alleles are selectively ablated in keratinocytes and which were obtained by crossing K14-Cre^{(tg/0)} transgenic mice *(LI et al., Development,* vol.128, p :675-688, 2001) with floxed VDR^{L2/L2} mice (our unpublished data)].

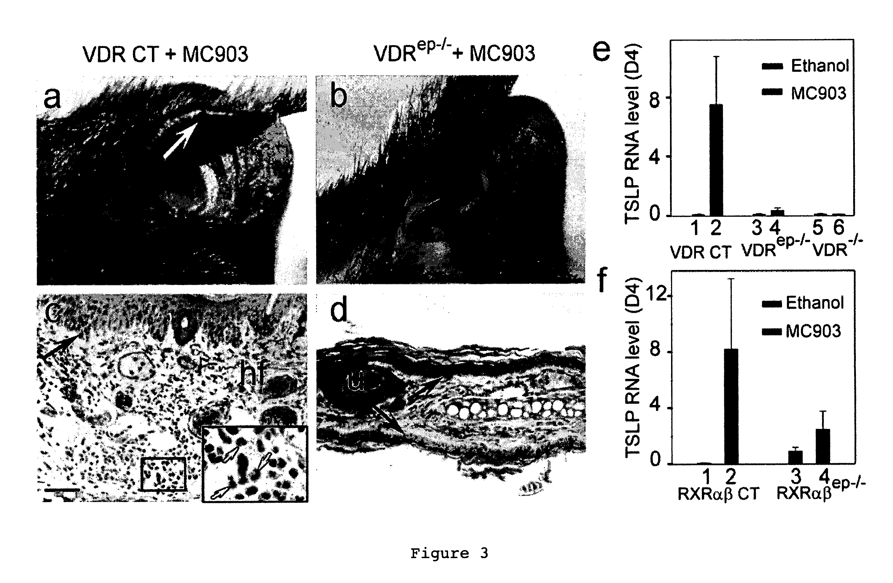

The Figures 3 a and b show the appearance of MC903-treated ears of VDR control (CT) (a) and VDR^{ep-/-} mice (b) at D 17. White arrows in (a) points to lesioned skin.

The Figures 3 c and d show Hematoxylin and eosin-stained ear sections. Yellow arrows in inset of (c) point to three of many eosinophils (red cytoplasmic staining) in MC903-treated CT skin. Black arrows point to the dermal/epidermal junction. hf, hair follicle; u, utriculi (resulting from hair follicle degeneration in VDR^{ep-/-} mice). (Scale bar, 50 µm).

Figure 3 e shows TSLP RNA levels (determined by Q-PCR) at D4 in ethanol- and MC903-treated ears of VDR CT (lane 1 and 2), VDR^{ep-/-} (lane 3 and 4) and VDR-/- (lane 5 and 6) mice.

The results show that at D17 of MC903 treatment, an AD-like inflammation was obvious on ears of VDR CT mice, whereas VDR^{ep-/-} ears did not show any sign of inflammation (see Fig. 3a and b). Accordingly, a massive dermal infiltrate of inflammatory cells, including eosinophils, CD4+ T helper cells, dendritic cells and mast cells, was detected in ear sections of MC903-treated VDR CT (see Fig. 3c, and data not shown), but not in those of VDR^{ep-/-} mice (see Fig. 3d). Similarly, no AD-like skin inflammation was developed upon topical MC903 treatment of VDR^{-/-} (germ line knockout) mice (YOSHIZAWA et al., Nat. Genet., vol.16, p :391-396, 1997 ; data not shown). TSLP expression (determined by Q-PCR) was strongly induced in MC903-treated skin of VDR CT mice (see Fig. 3e, lanes 1 and 2), but not at all in MC903-treated skin of VDR^{-/-} mice (lanes 5 and 6), whereas it was weakly increased in MC903-treated skin of VDR^{ep-/-} mutants (lanes 3 and 4). This latter increase may reflect a faint response to MC903 in non-keratinocytic cells. In any event, our data clearly demonstrated that induction of TSLP expression in keratinocytes upon MC903 application is a VDR-dependent cell-autonomous event that leads to the generation of an AD-like phenotype. In this respect, it is important to note that it has been recently reported that VDR-null mutant mice fail to develop symptoms of experimental asthma (WITTLE et al., J. Immunol., vol. 173, p: 3432-3436, 2004).

To examine whether the effect of MC903 was transduced through RXR/VDR heterodimers, ears of RXRαβ^{ep-/-} mice (tamoxifen-treated K14-Cre-ERT2^{(tg/0)}/RXRα^{L2/L2}/RXRβ^{L2/L2}; LI *et al.,* 2005, abovementioned), as well as their control littermates (RXRαβ CT), were topically-treated with MC903.

Figure 3 f shows TSLP RNA levels (determined by Q-PCR) at D4 in ethanol- and MC903-treated ears of RXRαβ CT (lane 1 and 2) and RXRαβ^{ep-/-} (lane 3 and 4) mice.

As expected *(LI et al.,* 2005, abovementioned), selective RXRαβ-ablation in keratinocytes of adult mice led to increased TSLP expression (lanes 1 and 3). However, the induction of TSLP by MC903 was severely reduced in RXRαβ^{ep-/-} skin (lanes 2 and 4), indicating an essential function of keratinocytic RXRs in TSLP induction by VDR agonists, most probably reflecting the involvement of RXR/VDR heterodimers.

### 4) VDR and RARγ agonistic ligands synergize to induce skin TSLP expression

Figure 1a shows that although much less potent than that of the active vitamin D3 [1α,25-(OH)2D3], application of a RARγ-selective agonist (BMS961) on mouse ear skin led to a significant increase in TSLP transcripts. A topical application of RA resulted in a similar induction (data not shown).

To examine whether VDR and RARγ agonists could synergize in upregulating TSLP expression, WT mouse ears were topically-treated for 3 days with either ethanol, BMS961 (4 nmoles), a limiting dose of 1α,25-(OH)2D3 (0.4 nmole), or a combination of the two ligands. Ear TSLP transcripts and serum TSLP were determined at D4.

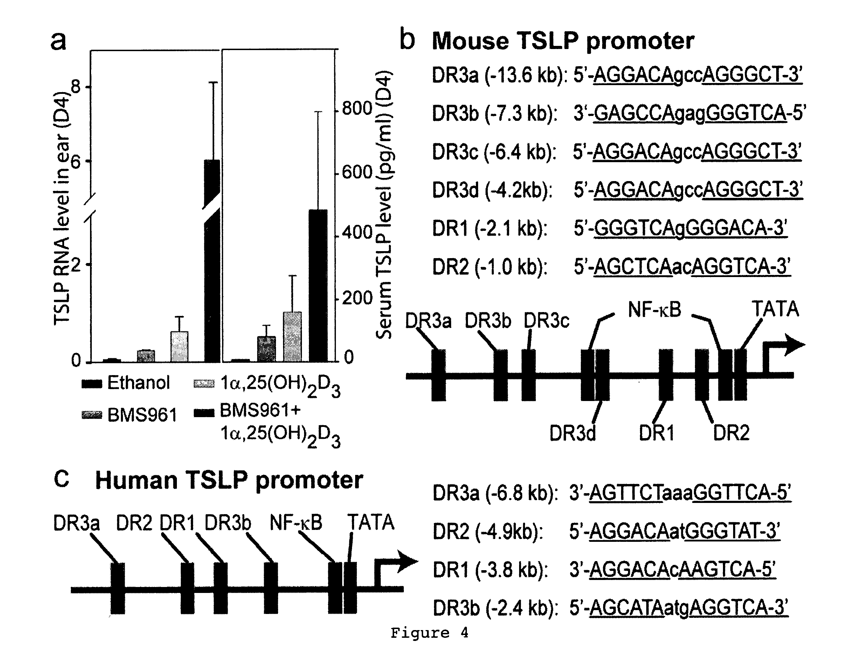

Figure 4 a shows TSLP RNA levels (determined by Q-PCR) in the ears (left panel) and serum TSLP levels (right panel) measured at D4 in mice ear-treated with either Ethanol, BMS961 (4 nmoles), 1α,25(OH)2D3 (0.4 nmoles) or BMS961 (4 nmoles) +1α,25(OH)2D3 (0.4 nmoles).

The results show a clear synergism between the effects of BMS961 and 1α,25-(OH)2D3 (see Fig. 4a), indicating a synergistic involvement of RARγ - and VDR-mediated events in transcriptional activation of TSLP expression. However, on its own, the induction of TSLP expression upon topical treatment with either BMS961 or RA, it too low to trigger an overt AD-like phenotype (data not shown).

Importantly, the fact that agonists of VDR and RARγ could induce TSLP expression either on their own or synergistically, indicates that the corresponding RXR heterodimers bind to distinct cognate response elements.

In this respect, it is noteworthy that both mouse and human TSLP promoter regions contain putative Response Elements (RE) (see Fig. 4b and c respectively) that may bind RXR/VDR heterodimers (VDRE: DR3) or RXR/RAR heterodimers (RARE: DR2 and DR1) (LEID et al., Trends Biochem. Sci., vol. 17, p :427-433, 1992).

Based on these evidence, the inventors propose a model accounting for the modulation of TSLP promoter activity by RXRα((β)/VDR and RXRα((β)/RARγ heterodimers.

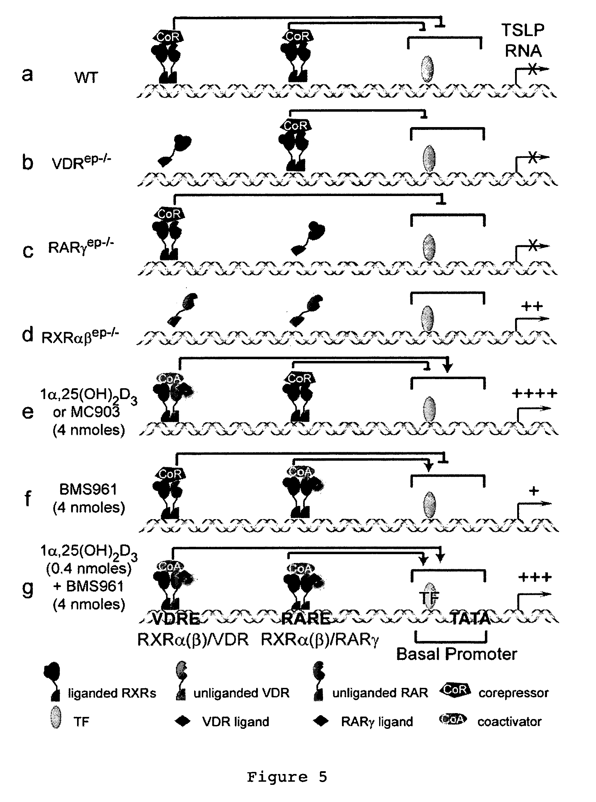

Figure 5 illustrates the schematic model of RXRα((β)/VDR- and RXRα(β)/RAR-mediated regulation of TSLP expression in mouse keratinocytes. The promoter region of mouse and human TLSP genes includes a TATA box element and proximal elements (e.g. NF-κB binding sites) (the basal promoter), as well as putative VDREs and RAREs (see Fig. 4b and c).

As under homeostatic conditions in vivo, there is no RA (Calléja et al., Genes & Dev. 20, p1525-1538, 2006) and very little, if any, active vitamin D3 in epidermal keratinocytes, the TSLP promoter basal activity is silenced by unliganded RXRα(β)/VDR and RXRα(β)/RARγ heterodimers associated with corepressors (PERISSI & ROSENFELD, Nat. Rev. Mol. Cell. Biol., vol.6, p :542-554, 2005; see Fig. 5a). This repression can be efficiently exerted by either RXRα(β)VDR or RXRα(β)/RARγ heterodimers, as it cannot be relieved by ablation of either VDR or RARγ (see Fig. 3e, and data not shown; Fig. 5b and c). On the other hand, RXRα and β ablation (see Fig. 5d), which releases both heterodimers from their binding sites, abolishes this repression and allows basal promoter-bound transcription factors to stimulate TSLP transcription to a basal activity (see Fig. 3f, lane 3) that is sufficient to trigger the generation of an AD-like phenotype (LI *et al.,* 2005, abovementioned).

Topical application of either active vitamin D3 or a low-calcemic analog (MC903) (see Fig. 5e) generates RXR/VDR-coactivator complexes whose transcriptional activity (see Fig. 3f, lane 2) is efficient enough to not only relieve the repression exerted by RXR/RARγ-corepressor complexes, but also to further enhance the basal promoter activity. Interestingly, the RXR/RARγ-coactivator complexes formed upon application of BMS961 are much less efficient (see Fig. 5f), as they generate lower TSLP transcript levels (see Fig. 1a) than those resulting from the basal promoter activity, as observed in keratinocytes ablated for RXRα and β (see Fig. 3f, lane 3). However, upon co-treatment with BMS961 and a limiting dose of 1α,25-(OH)₂D3 (see Fig. 5g), liganded RXR/RARγ and RXR/VDR heterodimers can efficiently synergize to enhance the activity of TSLP basal promoter (see Fig. 4a).

### 5) VDR and RAR antagonists down-regulate Vitamin D3-induced TSLP expression

The rapid and regulable induction of TSLP in mouse keratinocytes upon topical treatment with active Vitamin D3 or low-calcemic vitamin D3 analogs (e.g. MC903) provides a highly convenient AD preclinical model for exploring whether VDR and or RAR antagonists could be used to down-regulate the expression of TSLP.

Vitamin D3 antagonists ZK 168281 (2.5 nmoles) or TEI 9647 (2.5 nmoles) with Ethanol and with or without 1α,25(OH)₂D₃ (0.25 nmole) were daily topically-applied to ears of 6-8-week-old female CD1 (WT) mice. TSLP RNA levels in the ears were measured at day 4 (D4).

The results show that both of these Vitamin D3 antagonists down-regulate the 1α,25(OH)₂D₃ -induced TSLP expression (see. Figure 6).

The RAR antagonist BMS493 (2.5 nmoles) with ethanol and with or without MC903 (1.25 nmoles) was daily topically-applied to ears of 6-8-week-old female CD1 (WT) mice.TSLP RNA levels in the ears were measured at D3.

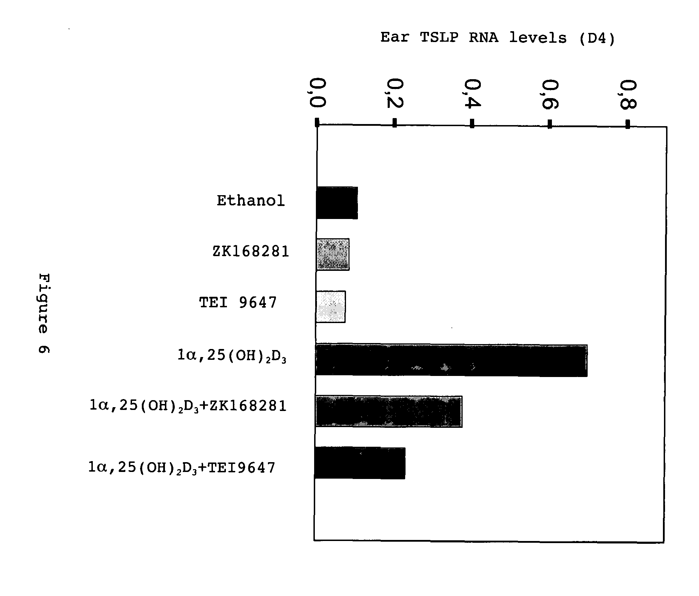

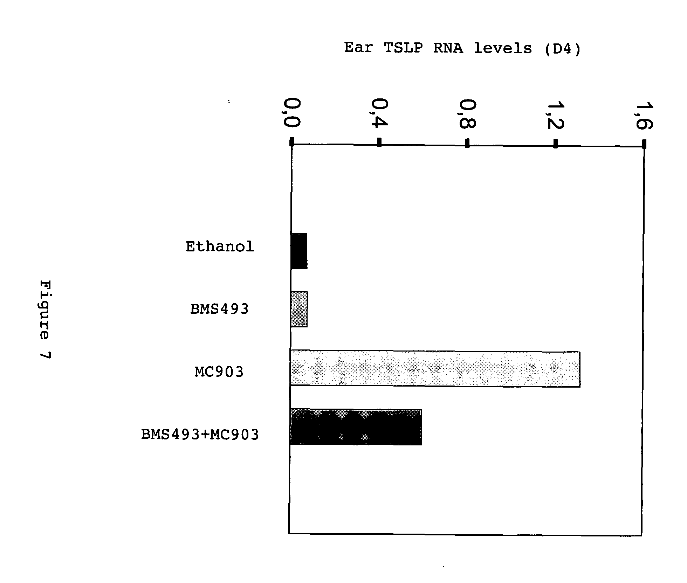

The results show that BMS493 down-regulates MC903-induced TSLP expression (see figure 7), indicating that an RXR/RARγ bound to a RAR antagonist can repress this induction of TSLP expression.

All documents referenced in this application, including those listed above, are herein incorporated by reference in their entirety. A variety of modifications to the embodiments described above will be apparent to those skilled in the art from the disclosure provided herein. Thus, the invention may be embodied in other specific forms without departing from the spirit or essential attributes thereof.

## Claims

1. A method for generating a human atopic disease-like phenotype in a mammal comprising the step (i) of administrating to said mammal at least one compound selected in the group comprising the physiologically active vitamin D3 (1α,25 (OH)₂ D₃) and agonistic analogs thereof.

2. The method according to claim 1, wherein said atopic disease is atopic dermatitis (AD).

3. The method according to claim 2, wherein said compound is administrated to the skin of said mammal.

4. The method according to claim 3, wherein said compound is administrated to the ear skin.

5. The method according to any of claims 2 to 4, further comprising the step of (ii) assessing the generation of atopic dermatitis like phenotype in said mammal.

6. The method according to claim 5, wherein said assessing step is realized by external skin aspect observation, skin histological examination, analyzing TSLP and Th2 type cytokine expression in skin, analyzing TSLP and IgE serum levels, and analyzing blood eosinophilia.

7. The method according to claim 1, wherein said atopic disease is asthma.

8. The method according to claim 7, wherein said compound is administrated to the lungs of said mammal.

9. The method according to claim 7, further comprising the step of (ii) assessing the generation of asthma in said mammal.

10. The method according to claim 9, wherein said assessing step is realized by lung histopathological examination, analysis of bonchoalveolar lavage (BAL) fluid, lung TSLP and Th2-type cytokine expression, and by physiological tests of lung function.

11. The method according to any of claims 1 to 10, wherein said mammal is a mouse.

12. The method according to any of claims 1 to 10, wherein said compound is the physiologically active vitamin D3 (1α,25 (OH)₂ D₃).

13. The method according to any of claims 1 to 10, wherein said compound is a vitamin D3 agonistic analog.

14. The method according to claim 13, wherein said vitamin D3 agonistic analog is selected in the group comprising 1α,18,25-(OH)₃D₃, 23-(m-(Dimethylhydroxymethyl)-22-yne-24,25,26,27(teranor)-1α-OH)₂D₃, 1α,25-Dihydroxy-trans-Isotachysterol(1,25-trans-Iso-T), (1S,3R,6S)-7,19-Retro-1,25-(OH)₂D₃, (1S,3R,6R)-7,19-Retro-1,25-(OH)₂D₃, 22-(p-(Hydroxyphenyl)-23,24,25,26,27-pentanor-D₃, 22-(m-(Hydroxyphenyl)-23,24,25,26,27-pentanor-D₃, 26, 27-cyclo-22-ene-1α,24S-dihydroxyvitamin D3 (MC903), 1(S),3(R)-dihydroxy-20(R)-(5'-ethyl-5'-hydroxy-hepta-1'(E),3'(E)-dien-1'-yl)-9,10-secopregna-5(Z),7(E),10(19)-triene (EB1089), 1α,25-(OH),-20-epi-22-oxa-24,26,27-trishomovitamin D (KH1060), and 1R,25-dihydroxy-21-(3-hydroxy-3-methylbutyl)vitaminD₃.

15. The method according to any of claims 13 or 14, wherein said vitamin D3 agonistic analog is a low-calcemic vitamin D3 agonistic analog selected in the group comprising 26, 27-cyclo-22-ene-1α,24S-dihydroxyvitamin D3 (MC903), 1(S),3(R)-dihydroxy-20(R)-(5'-ethyl-5'-hydroxy-hepta-1'(E),3'(E)-dien-1'-yl)-9,10-secopregna-5(Z),7(E),10(19)-triene (EB1089), and 1α,25-(OH),-20-epi-22-oxa-24,26,27-trishomovitamin D (KH1060), and 1R,25-dihydroxy-21-(3-hydroxy-3-methylbutyl)vitaminD₃.

16. The method according to any of claims 1 to 15, wherein the step (i) further comprises administrating to said mammal at least one compound selected in the group comprising natural and synthetic Retinoic Acid Receptor (RAR) agonists.

17. The method according to claim 16, wherein said compound is a synthetic RAR agonist selected in the group comprising the racemic mixture of R- and S-3-fluoro-4-[2-hydroxy-2-(5,5,8,8-tetramethyl-5,6,7,8,-tetrahydro-naphthalen-2-yl)-acetulamino]-benzoic acid (BMS961), (R)-3-Fluoro-4-[2-hydroxy-2-(5,6,7,8-tetrahydro-5,5,8,8-tetramethylnaphthalen-2-yl)-2-acetylamino]benzoic Acid (BMS270394), and (E)-4-[2-(5,6,7,8-Tetrahydro-5,5,8,8-tetramethylnaphthalen-2-yl)prop-1-en-1-yl]benzoic Acid (TTNPB).

18. The method according to any of claims 1 to 17, wherein said administration step is a daily administration and said administration step is maintained for a period comprised between 1 and 30 days, and can be resumed at any time thereafter.

19. The method according to any of claims 1 to 18, wherein said method is a method for identifying any compound that can be useful for treating and/or preventing an atopic disease, and wherein said method further comprises the step (iii) of administrating at least one of such compound to said mammal.

20. The method according to claim 19, wherein said compound that can be useful for treating and/or preventing an atopic disease is selected in the group comprising vitamin D3 antagonists and RAR antagonists.

21. The method according to claim 20, wherein said compound that can be useful for treating and/or preventing an atopic disease is a vitamin D3 antagonistic analog selected in the group comprising 14-Epi-1,25-(OH)₂D₃, 14-Epi-1,25-(OH)₂-Pre-D₃, 1,25-(OH)₂-7,8-cis-D₃, 1,25-(OH)₂-5,6-trans-7,8-cis-D₃, butyl-(5Z,7E,22E)-(1S,3R,24R)-1,3,24-trihydroxy-26,27-cyclo-9,10-secocholesta-5,7,10(19),22-tetraene-25-carboxylate (ZK159222), (23S)-25-dehydro-1α(OH)D3-26,23-lactone (TEI 9647) and ZK168281.

22. The method according to claim 20, wherein said compound that can be useful for treating and/or preventing an atopic disease is a vitamin D3 antagonistic analog that selectively antagonizes the effect of active vitamin D3 on immune system.

23. The method according to claim 20, wherein said compound that can be useful for treating and/or preventing an atopic disease is an RAR antagonist selected in the group comprising 4-(6-Methoxyethoxymethoxy-7-adamantyl naphthalen-2-yl)benzoic Acid (CD2665), 4-[2-(5,6-dihydro-5,5-dimethyl-8-p-tolylnaphthalen-2-yl)ethynyl]benzoic acid (AGN193109) and (E)-4-[2-[5,6-Dihydro-5,5-dimethyl-8-(2-phenylethynyl)naphthalene-2-yl]ethen-1-yl]benzoic Acid (BMS493).

24. The method according to any of claims 19 to 23, wherein said method further comprises the step (iv) of analyzing the human atopic disease-like phenotype of the mammal with or without the administration of the compound that has been identified to be possibly useful for treating and/or preventing a human atopic disease.

25. The method according to any of claims 19 to 24, wherein said method further comprises the step (v) of selecting the compounds that revert and/or prevent the human atopic disease-like phenotype.

26. Use of a composition comprising at least one vitamin D3 antagonist analog for the manufacture of a medicament for treating and/or preventing an atopic disease in a patient.

27. The use according to claim 26, wherein said atopic disease is atopic dermatitis (AD).

28. The use according to claim 27, wherein said vitamin D3 antagonist is a vitamin D3 antagonistic analog selected in the group comprising 14-Epi-1,25-(OH)₂D₃, 14-Epi-1,25-(OH)₂-Pre-D₃, 1,25-(OH)₂-7,8-cis-D₃, 1,25-(OH)₂-5,6-trans-7,8-cis-D₃, butyl-(5Z,7E,22E)-(1S,3R,24R)-1,3,24-trihydroxy-26,27-cyclo-9,10-secocholesta-5,7,10(19),22-tetraene-25-carboxylate (ZK159222), (23S)-25-dehydro-1α(OH)D3-26,23-lactone (TEI 9647), and ZK168281.

29. The use according to any of claims 26 or 27, wherein said vitamin D3 antagonistic analog selectively antagonizes the effect of active vitamin D3 on immune system.

30. The use according to any of claims 26 to 29, wherein said medicament is administrated to the skin of the patient.

31. The use according to any of claims 26 to 30, wherein the composition further comprises at least one RAR antagonist.

32. The use according to claim 31, wherein said RAR antagonist is selected in the group comprising 4-(6-Methoxyethoxymethoxy-7-adamantyl naphthalen-2-yl)benzoic Acid (CD2665), 4-[2-(5,6-dihydro-5,5-dimethyl-8-p-tolylnaphthalen-2-yl)ethynyl]benzoic acid (AGN193109) and (E)-4-[2-[5,6-Dihydro-5,5-dimethyl-8-(2-phenylethynyl)naphthalene-2-yl]ethen-1-yl]benzoic Acid (BMS493).

33. The use according to claim 26, wherein said atopic disease is asthma.

34. The use according to claim 33, wherein said medicament is administrated to the lungs
